# EUROPEAN PATENT SPECIFICATION

(11) **EP 3 711 601 B1**
(45) Date of publication and mention of the grant of the patent: **29.04.2026**
(21) Application number: 19164453.3
(22) Date of filing: 21.03.2019
(51) Int. Cl.: A61M 11/04, A61M 15/06, A24F 40/44, A24F 40/46, A61M 11/00, A24F 40/10

(54) **AEROSOL DELIVERY SYSTEM**
AEROSOLABGABESYSTEM
SYSTÈME D'ADMINISTRATION D'AÉROSOL

(43) Date of publication of application: 23.09.2020
(73) Proprietor: Imperial Tobacco Limited, Bristol, BS3 2LL (GB)
(72) Inventor: LORD, Chris, Speke, Liverpool L24 9HP (GB); SUDLOW, Thomas, Speke, Liverpool L24 9HP (GB); ILLIDGE, Ben, Speke, Liverpool L24 9HP (GB); MADDEN, Alfred, Speke, Liverpool L24 9HP (GB); ASTBURY, Ben, Speke, Liverpool L24 9HP (GB)
(74) Representative: Mewburn Ellis LLP

(56) References cited:
- EP-A1- 2 022 349
- WO-A1-2016/154798
- WO-A1-2018/019543
- WO-A1-2018/197511
- WO-A1-2019/042143
- CN-A- 101 843 368
- CN-A- 109 363 242
- CN-U- 206 079 033
- CN-U- 207 383 532
- US-A1- 2014 000 638
- US-A1- 2015 208 728
- US-A1- 2016 353 804
- US-A1- 2017 238 611
- US-A1- 2018 140 018
- US-A1- 2018 168 230

## Description

### Field of the Invention

The present invention relates to an aerosol delivery system comprising an aerosol-generation apparatus. The present invention relates to an aerosol delivery system including a heater configured to heat an aerosol precursor to generate an aerosolised composition for inhalation by a user, the aerosol delivery system comprising an aerosol-generation apparatus.

### Background

Pharmaceutical medicament, physiologically active substances and flavourings for example may be delivered to the human body by inhalation through the mouth and/or nose. Such material or substances may be delivered directly to the mucosa or mucous membrane lining the nasal and oral passages and/or the pulmonary system. For example, nicotine is consumed for therapeutic or recreational purposes and may be delivered to the body in a number of ways. Nicotine replacement therapies are aimed at people who wish to stop smoking and overcome their dependence on nicotine. Nicotine is delivered to the body in the form of aerosol delivery devices and systems, also known as smoking-substitute devices or nicotine delivery devices. Such devices may be non-powered or powered.

Devices or systems that are non-powered may comprise nicotine replacement therapy devices such as "inhalators", e.g. Nicorette^{®} Inhalator. These generally have the appearance of a plastic cigarette and are used by people who crave the behaviour associated with consumption of combustible tobacco - the so-called hand-to-mouth aspect - of smoking tobacco. Inhalators generally allow nicotine-containing aerosol to be inhaled through an elongate tube in which a container containing a nicotine carrier, for example, a substrate, is located. An air stream caused by suction through the tube by the user carries nicotine vapours into the lungs of the user to satisfy a nicotine craving. The container may comprise a replaceable cartridge, which includes a cartridge housing and a passageway in the housing in which a nicotine reservoir is located. The reservoir holds a measured amount of nicotine in the form of the nicotine carrier. The measured amount of nicotine is an amount suitable for delivering a specific number of "doses". The form of the nicotine carrier is such as to allow nicotine vapour to be released into a fluid stream passing around or through the reservoir. This process is known as aerosolization and or atomization. Aerosolization is the process or act of converting a physical substance into the form of particles small and light enough to be carried on the air i.e. into an aerosol. Atomization is the process or act of separating or reducing a physical substance into fine particles and may include the generation of aerosols. The passageway generally has an opening at each end for communication with the exterior of the housing and for allowing the fluid stream through the passageway. A nicotine-impermeable barrier seals the reservoir from atmosphere. The barrier includes passageway barrier portions for sealing the passageway on both sides of the reservoir. These barrier portions are frangible so as to be penetrable for opening the passageway to atmosphere.

A device or a system that is powered can fall into two sub-categories. In both subcategories, such devices or systems may comprise electronic devices or systems that permit a user to simulate the act of smoking by producing an aerosol mist or vapour that is drawn into the lungs through the mouth and then exhaled. The electronic devices or systems typically cause the vaporization of a liquid containing nicotine and entrainment of the vapour into an airstream. Vaporization of an element or compound is a phase transition from the liquid phase to vapour i.e. evaporation or boiling. In use, the user experiences a similar satisfaction and physical sensation to those experienced from a traditional smoking or tobacco product, and exhales an aerosol mist or vapour of similar appearance to the smoke exhaled when using such traditional smoking or tobacco products.

A person of ordinary skill in the art will appreciate that devices or systems of the second, powered category as used herein include, but are not limited to, electronic nicotine delivery systems, electronic cigarettes, e-cigarettes, e-cigs, vaping cigarettes, pipes, cigars, cigarillos, vaporizers and devices of a similar nature that function to produce an aerosol mist or vapour that is inhaled by a user. Such nicotine delivery devices or systems of the second category incorporate a liquid reservoir element generally including a vaporizer or misting element such as a heating element or other suitable element, and are known, inter alia, as atomizers, cartomizers, or clearomizers. Some electronic cigarettes are disposable; others are reusable, with replaceable and refillable parts.

Aerosol delivery devices or systems in a first sub-category of the second, powered category generally use heat and/or ultrasonic agitation to vaporize a solution comprising nicotine and/or other flavouring, propylene glycol and/or glycerine-based base into an aerosol mist of vapour for inhalation.

Aerosol delivery devices or systems in a second sub-category of the second, powered category may typically comprise devices or systems in which tobacco is heated rather than combusted. During use, volatile compounds may be released from the tobacco by heat transfer from the heat source and entrained in air drawn through the aerosol delivery device or system. Direct contact between a heat source of the aerosol delivery device or system and the tobacco heats the tobacco to form an aerosol. As the aerosol containing the released compounds passes through the device, it cools and condenses to form an aerosol for inhalation by the user. In such devices or systems, heating, as opposed to burning, the tobacco may reduce the odour that can arise through combustion and pyrolytic degradation of tobacco.

Aerosol delivery devices or systems falling into the first sub-category of powered devices or systems may typically comprise a powered unit, comprising a heater element, which is arranged to heat a portion of a carrier that holds an aerosol precursor. The carrier comprises a substrate formed of a "wicking" material, which can absorb aerosol precursor liquid from a reservoir and hold the aerosol precursor liquid. Upon activation of the heater element, aerosol precursor liquid in the portion of the carrier in the vicinity of the heater element is vaporised and released from the carrier into an airstream flowing around the heater and carrier. Released aerosol precursor is entrained into the airstream to be borne by the airstream to an outlet of the device or system, from where it can be inhaled by a user.

The heater element is typically a resistive coil heater, which is wrapped around a portion of the carrier and is usually located in the liquid reservoir of the device or system. Consequently, the surface of the heater may always be in contact with the aerosol precursor liquid, and long-term exposure may result in the degradation of either or both of the liquid and heater. Furthermore, residues may build up upon the surface of the heater element, which may result in undesirable toxicants being inhaled by the user. Furthermore, as the level of liquid in the reservoir diminishes through use, regions of the heater element may become exposed and overheat.

The present invention has been devised in light of the above considerations.

US 2018/0140018 A1 discloses an aerosol generator, an electronic cigarette, and a detachable atomizing device.

US 2016/353804 A1 discloses an electronic vapour provision device.

US 2015/208728 A1 discloses an electronic vapour provision device.

WO 2019/042143 A1 discloses an aerosol generating device and system.

WO 2016/154798 A1 discloses an atomizer and electronic cigarette.

CN 101843368 A discloses a section nozzle for an electronic atomizer.

CN 207383532 U discloses an electronic cigarette and atomizing component thereof.

CN 206079033 U discloses a low temperature cigarette.

### Summary of the Invention

The invention is defined by the claims.

At its most general, the present disclosure proposes that an aerosol-generation apparatus has a fluid-transfer article with a bore therein which receives at least part of a heater, which is referred to herein as a penetrating part of the heater. The wall of the bore forms an activation surface, and the fluid-transfer article holds an aerosol precursor and transfers that aerosol precursor to the activation surface. The activation surface varies in spacing from the penetrating part of the heater, with one or more air-flow pathways are defined between the activation surface and the penetrating part of the heater.

The penetrating part of the heater may heat aerosol precursor at the activation surface, and release it from that surface as vapour and/or an aerosol and vapour mixture, which vapour or mixture then passes in to the air-flow pathway or pathways, from whence it may pass to a user. Since the bore, and hence the activation surface, is within the fluid-transfer article, escape of aerosol precursor when the heater is not active may be minimized.

Thus, according to the present disclosure, there may be provided an aerosol-generation apparatus comprising a heater and a fluid-transfer article, the fluid-transfer article being for holding an aerosol precursor and transferring the aerosol precursor to an activation surface of said fluid-transfer article, the fluid-transfer article having a bore therein within which is a least a penetrating part of the heater, within which at least a penetrating of the wall of the bore surrounding the penetrating part of the heater and forming said activation surface, the activation surface having a varying spacing from said penetrating part, thereby to define at least one air-flow pathway between said activation surface and said penetrating part of said heater.

Preferably, at least one part of the wall of the bore is in contact with said penetrating part of said heater, and at least another part of said bore defines at least one channel between the wall of the bore and the penetrating part of the heater, said at least one air-flow pathway being along said at least one channel. In such an arrangement, there may be a plurality of channels around the penetrating part of the heater.

For example, they may be uniformly spaced around the penetrating part.

Preferably said penetrating part is elongate in the direction of the axis of the bore. Then, in such an arrangement, the channel or channels will normally extend in a direction parallel to the direction of elongation of the penetrating part of the heater within the bore. Thus, the channel or channels extend along the penetrating part of the heater, e.g. from one end to the other, to maximize the amount of the activation surface which is close to the heater.

Normally, the penetrating part of the heater is cylindrical, so it conforms to the bore in the fluid-transfer article. However, other cross-sectional shapes are possible, such as square or triangular, particularly if the bore has a similar shape. It would also be possible for the penetrating part to be tapered, which may facilitate its insertion into the bore.

The fluid-transfer article may have a first region surrounding the penetrating part of the heater, with a surface of that first region forming the activation surface. That first region thus transfers aerosol precursor to the activation surface. It may therefore be formed of a porous polymer material, or of a fibrous material, or any other material which has a "wicking" action to facilitate transfer of aerosol precursor.

The penetrating part may be the only active part of the heater, so that heat is only generated within the bore itself. Alternatively, the heater may have a base part, from which the penetrating part extends. The base part may then be adjacent the end of the fluid-transfer article, so that the fluid-transfer article receives heat from both the penetrating part and the base part.

The aerosol-generation apparatus may form part of an aerosol delivery system, which has a housing containing the fluid-transfer article. There may be a further housing which supports the heater, with the housing and further housing being separable. The aerosol precursor in the fluid-transfer article will be consumed, and need to be replaced. Whilst this could be achieved by re-filling the fluid-transfer article while it is in situ, it is preferable that the carrier is removed by separating the housing and the further housing, to enable the fluid-transfer article to be re-filled separately, or replaced.

The disclosure includes the combination of the aspects and preferred features described except where such a combination is clearly impermissible or expressly avoided.

### Summary of the Figures

So that the invention may be understood, and so that further aspects and features thereof may be appreciated, embodiments illustrating the principles of the invention will now be discussed in further detail with reference to the accompanying figures, in which:
**Figure 1** is a perspective view illustration of a system for aerosol delivery according to one or more embodiments of the present invention;
**Figure 2** is a cross-section side view illustration of part of an apparatus of the system for aerosol delivery of Figure 1;
**Figure 3** is a cross-section side view illustration of the system and apparatus for aerosol delivery of Figure 1;
**Figure 4** is a perspective view illustration of an aerosol carrier for use in the system for aerosol delivery according to one or more embodiments of the present invention;
**Figure 5** is a cross-sectional view of the aerosol carrier of Figure 4, along the line A-A in Figure 4, but with the penetrating part of the heater also illustrated;
**Figure 6** is a cross-section side view of an aerosol carrier for use in an aerosol delivery system according to one or more embodiments of the present invention, along the line B-B in Figure 5;
**Figure 7** is a perspective cross-section side view of the aerosol carrier of Figure 7;
**Figure 8** is a cross-section side view of the aerosol carrier for use in an aerosol delivery system according to one or more embodiments of the present invention along the line C-C in Figure 5;
**Figure 9** is a perspective cross-section side view of the aerosol carrier shown in Figure 8, and;
**Figure 10** is an exploded perspective view illustration of a kit-of-parts for assembling the system according to one or more embodiments of the present invention.

### Detailed Description of the Invention

Aspects and embodiments of the present invention will now be discussed with reference to the accompanying figures. Further aspects and embodiments will be apparent to those skilled in the art.

In general outline, one or more embodiments in accordance with the present disclosure may provide a system for aerosol delivery in which an aerosol carrier may be inserted into a receptacle (e.g. a "heating chamber") of an apparatus for initiating and maintaining release of an aerosol from the aerosol carrier. Another end, or another end portion, of the aerosol carrier may protrude from the apparatus and can be inserted into the mouth of a user for the inhalation of aerosol released from the aerosol carrier cartridge during operation of the apparatus.

Hereinafter, and for convenience only, "system for aerosol delivery" shall be referred to as "aerosol delivery system".

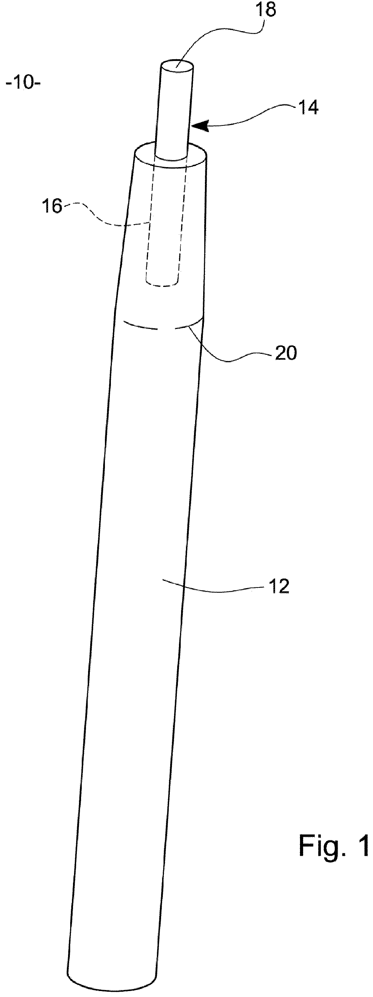

Referring now to Figure 1, there is illustrated a perspective view of an aerosol delivery system 10 comprising an aerosol generation apparatus 12 operative to initiate and maintain release of aerosol from a fluid-transfer article in an aerosol carrier 14. In the arrangement of Figure 1, the aerosol carrier 14 is shown with a portion of the length of the aerosol carrier 14 located within a receptacle of the apparatus 12. A remaining portion of the aerosol carrier 14 extends out of the receptacle. This remaining portion of the aerosol carrier 14, terminating at a second end 18 of the aerosol carrier, is configured for insertion into a user's mouth. A vapour and/or aerosol is produced when a heater (not shown in Figure 1) of the apparatus 12 heats a fluid-transfer article in the aerosol carrier 14 to release a vapour and/or an aerosol, and this can be delivered to the user, when the user sucks or inhales, via a fluid passage in communication with an outlet of the aerosol carrier 14 from the fluid-transfer article to the second end 18.

The device 12 also comprises air-intake apertures 20 in the housing of the apparatus 12 to provide a passage for air to be drawn into the interior of the apparatus 12 (when the user sucks or inhales) for delivery to the first end 16 of the aerosol carrier 14, so that the air can be drawn across an activation surface of a fluid-transfer article located within a housing of the aerosol carrier cartridge 14 during use. Optionally, these apertures may be perforations in the housing of the apparatus 12.

A fluid-transfer article (not shown in Figure 1) is located within a housing of the aerosol carrier 14. The fluid-transfer article contains an aerosol precursor material, which may include at least one of: nicotine; a nicotine precursor material; a nicotine compound; and one or more flavourings. The fluid-transfer article is located within the housing of the aerosol carrier 14 to allow air drawn into the aerosol carrier 14 at, or proximal, the first end 16 to flow across an activation surface of the fluid-transfer article. As air passes across the activation surface of the fluid-transfer article, an aerosol may be entrained in the air stream from a substrate forming the fluid-transfer article, e.g. via diffusion from the substrate to the air stream and/or via vaporisation of the aerosol precursor material and release from the fluid-transfer article under heating.

The aerosol carrier 14 is removable from the apparatus 12 so that it may be disposed of when expired. After removal of a used aerosol carrier 14, a replacement aerosol carrier 14 can be inserted into the apparatus 12 to replace the used aerosol carrier 14.

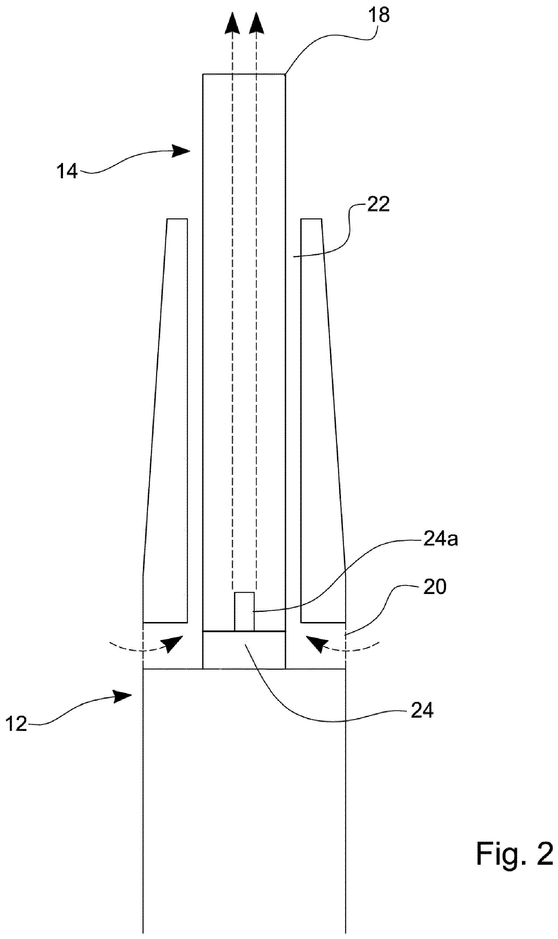

Figure 2 is a cross-sectional side view illustration of a part of apparatus 12 of the aerosol delivery system 10. The apparatus 12 comprises a receptacle 22 in which is located a portion of the aerosol carrier 14. In one or more optional arrangements, the receptacle 22 may enclose the aerosol carrier 14. The apparatus 12 also comprise a heater 24, which will be described in more detail later.

Air flows into the apparatus 12 (in particular, into a closed end of the receptacle 22) via air-intake apertures 20. From the closed end of the receptacle 22, the air is drawn into the aerosol carrier 14 (under the action of the user inhaling or sucking on the second end 18) and expelled at the second end 18. As the air flows into the aerosol carrier 14, it passes across the activation surface of the fluid-transfer article. Heat from the heater 24, causes vaporisation of aerosol precursor material at the activation surface of the fluid-transfer article and an aerosol is created in the air flowing over the activation surface. Thus, through the application of heat in the region of the activation surface of the fluid-transfer article, an aerosol is released, or liberated, from the fluid-transfer article, and is drawn from the material of the aerosol carrier unit by the air flowing across the activation surface and is transported in the air flow to via outlet conduits (not shown in Figure 2) in the housing of the aerosol carrier 14 to the second end 18. The direction of air flow is illustrated by arrows in Figure 2.

To achieve release of the captive aerosol from the fluid-transfer article, the fluid-transfer article of the aerosol carrier 14 is heated by the heater 24. As a user sucks or inhales on second end 18 of the aerosol carrier 14, the aerosol released from the fluid-transfer article and entrained in the air flowing across the activation surface of the fluid-transfer article is drawn through the outlet conduits (not shown) in the housing of the aerosol carrier 14 towards the second end 18 and onwards into the user's mouth.

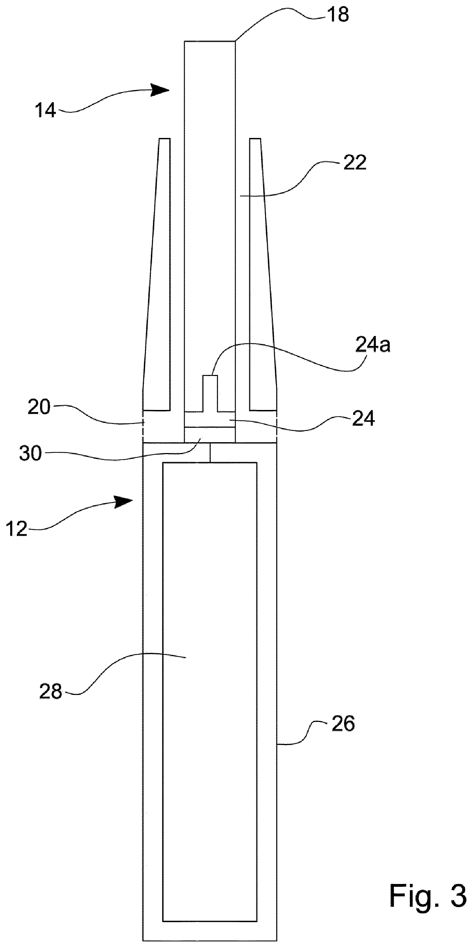

Turning now to Figure 3, a cross-sectional side view of the aerosol delivery system 10 is schematically illustrated showing the features described above in relation to Figures 1 and 2 in more detail. As can be seen, apparatus 12 comprises a housing 26, in which are located the receptacle 22 and heater 24. The housing 26 also contains control circuitry (not shown) operative by a user, or upon detection of air and/or vapour being drawn into the device 12 through air-intake apertures 20, i.e. when the user sucks or inhales. Additionally, the housing 26 comprises an electrical energy supply 28, for example a battery. Optionally, the battery comprises a rechargeable lithium ion battery. The housing 26 also comprises a coupling 30 for electrically (and optionally mechanically) coupling the electrical energy supply 28 to control circuitry (not shown) for powering and controlling operation of the heater 24. A penetrating part 24a of the heater 24 extends into the receptacle 22 as will subsequently be described.

Responsive to activation of the control circuitry of apparatus 12, the heater 24 heats the fluid-transfer article (not shown in Figure 3) of aerosol carrier 14. This heating process initiates (and, through continued operation, maintains) release of vapour and/or an aerosol from the activation surface of the fluid-transfer article. The vapour and/or aerosol formed as a result of the heating process is entrained into a stream of air being drawn across the activation surface of the fluid-transfer article (as the user sucks or inhales). The stream of air with the entrained vapour and/or aerosol passes through the aerosol carrier 14 via outlet conduits (not shown) and exits the aerosol carrier 14 at second end 18 for delivery to the user. This process is briefly described above in relation to Figure 2, where arrows schematically denote the flow of the air stream into the device 12 and through the aerosol carrier 14, and the flow of the air stream with the entrained vapour and/or aerosol through the aerosol carrier cartridge 14.

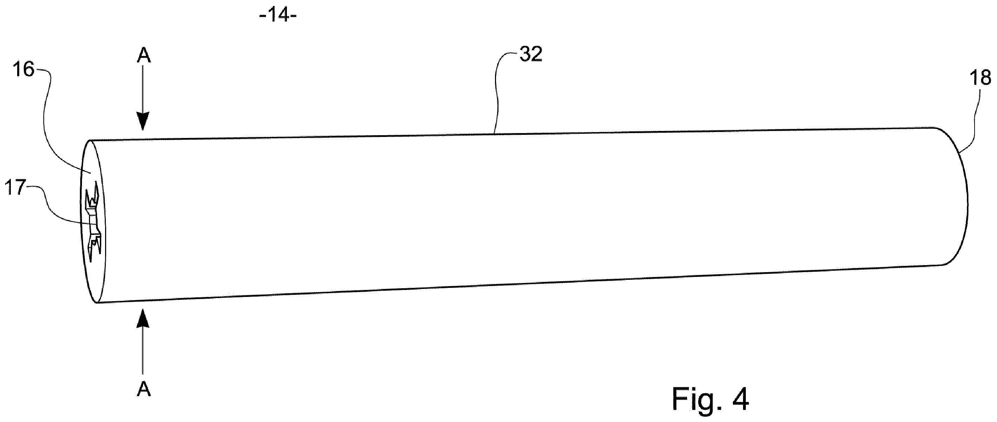

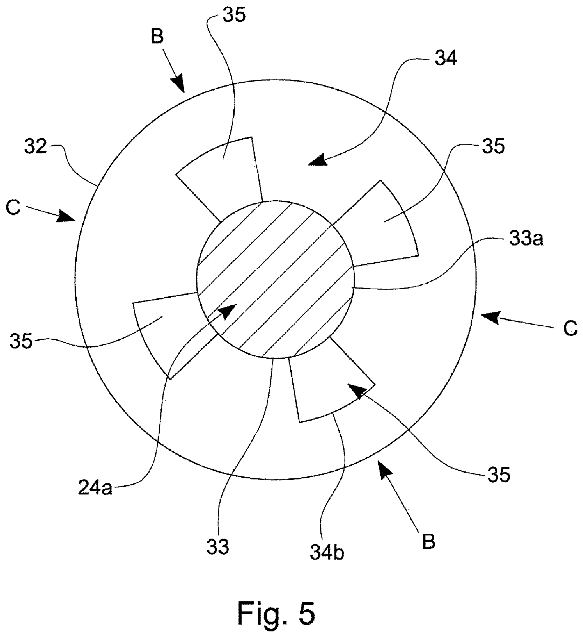

Figures 4 to 5 schematically illustrate the aerosol carrier 14 in more detail (and, in Figure 5, features within the receptacle in more detail). Figure 4 illustrates an exterior of the aerosol carrier 14, Figure 5 illustrates internal components of the aerosol carrier 14 in one optional configuration.

Fig. 4 illustrates the exterior of the aerosol carrier 14, which comprises a housing 32 for housing said fluid- transfer article (not shown) and at least one other internal component. The particular housing 32 illustrated in Figure 4 comprises a tubular member, which may be generally cylindrical in form, and which is configured to be received within the receptacle of the apparatus. First end 16 of the aerosol carrier 14 is for location to oppose the heater of the apparatus, and second end 18 (and the region adjacent the second end 18) is configured for insertion into a user's mouth.

Further components not shown in Figure 4 comprise: an inlet conduit, via which air can be drawn into the aerosol carrier 14; an outlet conduit, via which an air stream entrained with aerosol can be drawn from the aerosol carrier 14; a filter element; and a reservoir for storing aerosol precursor material.

Figure 4 also shows that one end 16 of the housing 32 has a hole 17 therein. The end 16 is solid to limit escape of aerosol precursor from the carrier 14. The shape of the hole 17 will be described in more detail with reference to Figure 5.

Figure 5 illustrates a cross-section view through housing 32, along the line A-A in Figure 4, but in which the penetrating part 24a of the heater 24 inserted into the end of the carrier 14, so that the operation of the apparatus may be described.

Thus, Figure 5 shows that the housing 32 contains a fluid-transfer article having a first region 34 holding an aerosol precursor. The first region 34 may fill the housing 32, so that it acts as the reservoir for the aerosol precursor, or there may be a separate reservoir within the housing 32. The first region 34 of the fluid-transfer article has a bore 33 therein, into which bore is inserted the penetrating part 24a of the heater. The wall of the first region 34 of the fluid-transfer article which defines the periphery of the bore 33 is not circular. Instead, it comprises arc regions 33a in contact with the penetrating part 24a of the heater 24, and regions 34b spaced from the penetrating part 24a of the heater 24. Thus, channels 35 are formed around the penetrating part of the heater 24, at the circumferentially spaced apart regions 34b.

The first region 34 of the fluid-transfer article acts to transfer aerosol precursor to the bore 33, so that the wall of the bore 33 (and in particular the part of the wall of the bore 33 forming the region 34b) acts as an activation surface. The activation surface, and adjacent parts of the first region 34 of the fluid-transfer article will be heated when the heater 24 is active, by transfer of heat from the penetrating part 24a of the heater 24. This causes the aerosol precursor to vaporise and the vapour, or a mixture of aerosol and vapour, will be released from the activation surface into the channels 35. As will be described later, those channels 35 act as air-flow pathways within the carrier 14, when the apparatus is being used, and thus the vapour or aerosol/vapour mixture will mix with the air flowing in the channels 35, and may then pass to the user. It should be noted that the hole 17 in the end 16 of the carrier 14 will conform to the configuration of the bore 33 within the first part 34 of the fluid-transfer article. Thus, the parts of the hole 17 corresponding to the channels 35 will act as inlets to those channels 35.

The first part 34 of the fluid-transfer article may be a porous material where pores of the porous material hold, contain, carry, or bear the aerosol precursor material. In particular, the porous material of the fluid-transfer article may be a porous polymer material such as, for example, a sintered material. Particular examples of material suitable for the fluid-transfer article include: Polyetherimide (PEI); Polytetrafluoroethylene (PTFE); Polyether ether ketone (PEEK); Polyimide (PI); Polyethersulphone (PES); and Ultra-High Molecular Weight Polyethylene. Other suitable materials may comprise, for example, BioVyonTM (by Porvair Filtration Group Ltd) and materials available from Porex^{®}. Further optionally, a substrate forming the fluid-transfer article may comprise Polypropylene (PP) or Polyethylene Terephthalate (PET). All such materials may be described as heat resistant polymeric wicking material in the context of the present invention.

The present invention is not limited to the use of such polymeric wicking material, and other wicking materials may be used. For example, the first part 34a of the fluid-transfer article may be of fibrous material, or any other known wicking material.

Referring again to Figure 2, the air-intake apertures 20 communicate with the hole 17, and the ends of the channels 35 communicate with second end 18 of the carrier 14. Thus, when the user sucks on the end 18, air will be drawn into the air-intake apertures 20, through the hole 17 along the channels 35. If the heater 24 is active, the aerosol precursor at the activation surface will be released as vapour or aerosol/vapour mixture, and will then pass to the end 18.

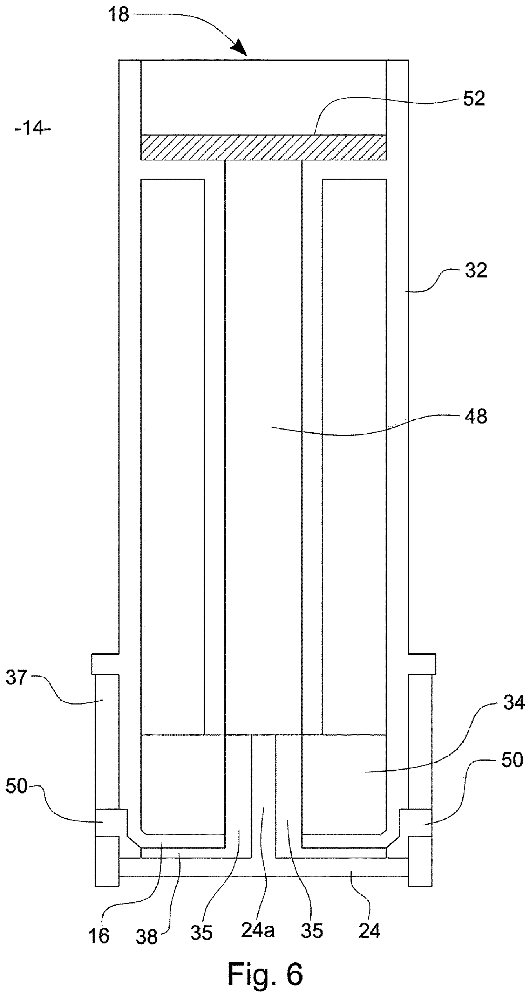

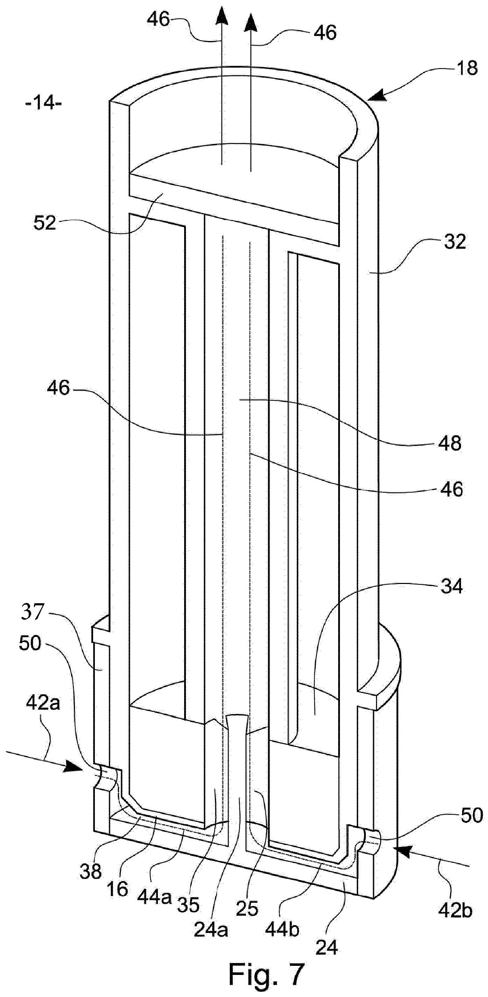

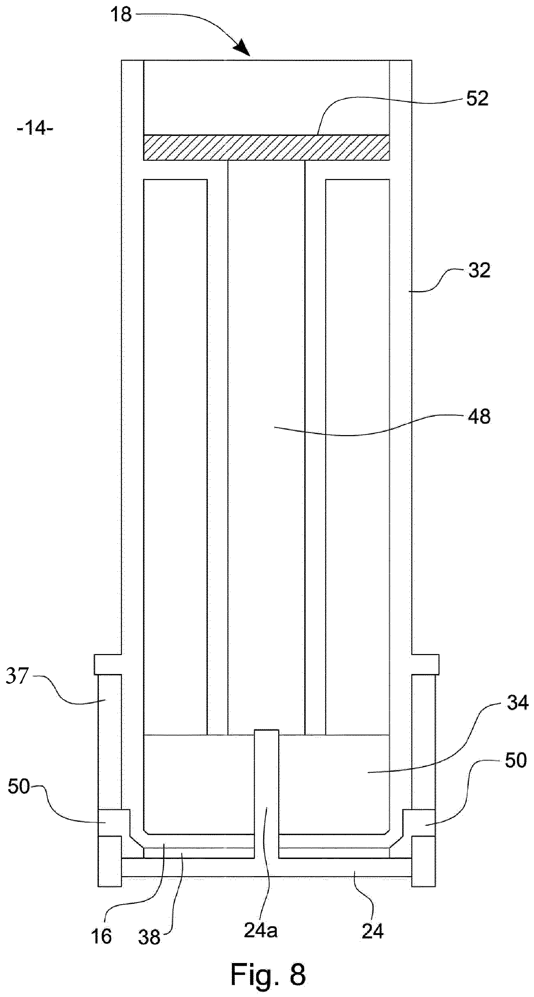

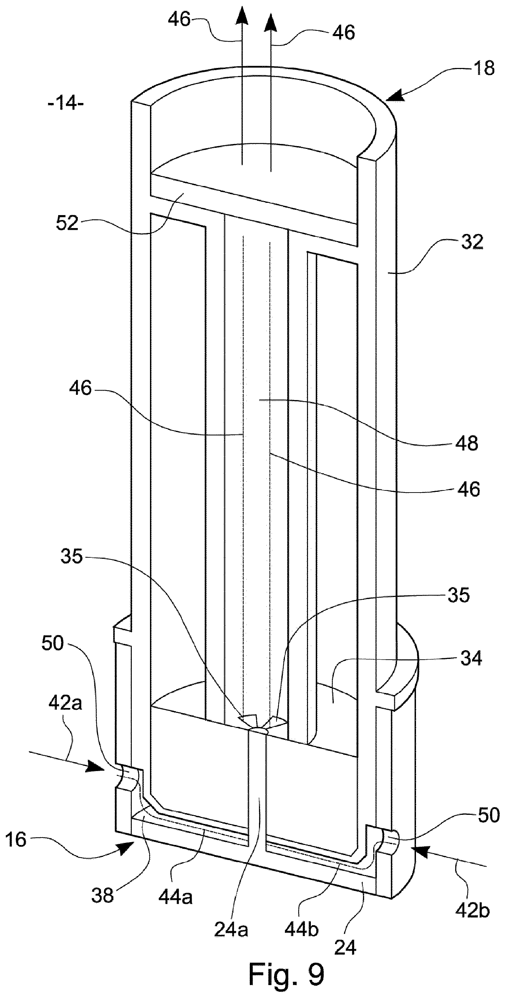

The structure of the aerosol carrier 14 and adjacent parts of the rest of the apparatus are illustrated in Figures 6 to 9. Figures 6 and 7 correspond to the views along the line B-B in Figure 5, whereas Figures 8 and 9 correspond to the views along the line C-C.

As can be seen from Figures 6 to 9, the particular configuration of aerosol carrier 14 illustrated is generally tubular in form. The aerosol carrier 14 comprises the housing 32, which defines the external walls of the aerosol carrier 14 and which defines therein a chamber in which are disposed the fluid-transfer article 34 (adjacent the first end 16 of the aerosol carrier 14) and internal walls defining the fluid communication pathway 48. Fluid communication pathway 48 defines a fluid pathway for an outgoing air stream from the channels 40 to the second end 18 of the aerosol carrier 14. In the examples illustrated in Figures 6 to 9, the fluid-transfer article 34 is an annular shaped element located around the fluid communication pathway 48.

Figure 6 to 9 also illustrates a further housing 37, which further housing supports the heater 24, into which the housing 32 is fitted, with the housing 32 and the further housing 37 being separable. Figures 6 to 9 also illustrate that, when the housings 32 and 37 are fitted together, there is a gap 38 between the end 16 of the carrier 14 and the heater 24. That gap 38 allows air to flow which will be described later. The further housing 37 may be integral with the housing 26 containing the electrical energy supply 28.

In walls of the further housing 37, there are provided inlet apertures 50 to provide a fluid communication pathway for an incoming air stream to reach the fluid-transfer article, and particularly the air-flow pathways defined between the activation surface of the fluid-transfer article and the penetrating part 24a of the heater 24, along the channels 35.

In the illustrated example of Figures 6 and 7, the aerosol carrier 14 further comprises a filter element 52. The filter element 52 is located across the fluid communication pathway 48 such that an outgoing air stream passing through the fluid communication pathway 48 passes through the filter element 52.

With reference to Figure 7, when a user sucks on a mouthpiece of the apparatus (or on the second end 18 of the aerosol carrier 14, if configured as a mouthpiece), air is drawn into the carrier through inlet apertures 50 extending through walls in the housing 32 of the aerosol carrier 14.

An incoming air stream 42a from a first side of the aerosol carrier 14 passes between the end 16 of the carrier 14 and the heater 24, through gap 38 to form an airstream 44a which passes to the hole 17, and hence to the channels 35. Similarly, an incoming airstream 42b from a second side of the aerosol carrier generally passes between the end 16 of the carrier 14 and the heater 24 through gap 38, to form an airstream 44b, which also passes through the hole 17 to the passageways 35. These airstreams from each side are denoted by dashed lines 44a and 44b in Figure 7.

In use, the heater 24 of the apparatus 12, and in particular the penetrating part 24a of the heater 24 raise a temperature of the first part 34 of the fluid-transfer article at the activation surface to a sufficient temperature to release, or liberate, captive substances (i.e. the aerosol precursor) to form a vapour and/or aerosol, which is drawn downstream. As the air streams 44a and 44b continue their passages into the channels 35, more released aerosol precursor is entrained within the air streams 44a and 44b. When the air streams 44a and 44b entrained with aerosol precursor meet at a mouth of the outlet fluid communication pathway 48, they enter the outlet fluid communication pathway 48 and continue until they pass through filter element 52 and exit outlet fluid communication pathway 48, either as a single outgoing air stream, or as separate outgoing air streams 46 (as shown). The outgoing air streams 46 are directed to an outlet, from where it can be inhaled by the user directly (if the second end 18 of the aerosol capsule 14 is configured as a mouthpiece), or via a mouthpiece. The outgoing air streams 46 entrained with aerosol precursor are directed to the outlet (e.g. via a gas communication pathway within the housing of the carrier).

Figures 8 and 9 then correspond to the views of Figures 6 and 7, but along the line C-C in Figure 5. Thus, in Figures 8 and 9, the first part 34 of the fluid-transfer article makes contact with the penetrating part 24a of the heater 24. Channels 35 are visible in Figure 9, in a perspective view.

Note that it is possible for there to be space between the first part 34 of the fluid-transfer article and the penetrating part 24a of the heater 24 at regions other than the channels 35, such as at the arc regions 33a. However, contact at those arc regions 33a is preferred since it will maximise heat transfer. The size and number of the channels 35 will need to be chosen to permit sufficient heat to be transferred from the penetrating part 24a of the heater 24 to the first part 34 of the fluid transfer article, but also to provide sufficiently large channels 35 for the air flow to be suitable for the user. In the embodiments described with reference to Figures 5 to 9 there are four channels 35 spaced uniformly around the penetrating part 24a of the heater 24. More or fewer channels 35 may be provided if desired.

As mentioned above, the housings 32 and 37 are separable. As the user uses the apparatus, the aerosol precursor in the carrier 14 will be consumed and will need to be replaced. To do this, it is easiest if the housing 32 and 37 are separated, and the carrier 14 removed and replaced. The replaced carrier may be re-filled or discarded. Whatever the case, a carrier containing fluid-transfer article filled with aerosol precursor will need to be mounted so that its housing 32 is fitted into the housing 37, as illustrated in Figures 6 to 9, but the user may continue to use the apparatus. It should be noted that the penetrating part 24a of the heater 24 remains with the housing 37, so the heater 24 does not need to be replaced. For this reason, the first part 34a of the fluid-transfer article is not attached to the penetrating part 24a of the heater 24. Figure 5 illustrates an abutting unbonded contact therebetween.

When the carrier 14 is removed from the rest of the apparatus, if there is residual aerosol precursor in the first part 34 of the fluid-transfer article, end 16 of the carrier 14 will prevent or limit escape of the residual aerosol precursor, as the end 16 is closed except at the hole 17 by part of the housing 32.

In the arrangement of Figures 6 to 9, the penetrating part 24a extends form the rest of the heater 24, with the rest of the heater 24 forming a bore which bounds the gaps 38. This facilitates the heating of the air before it reaches the channels 35.

In the embodiment of Figures 5 to 9, the penetrating part 24a of the heater 24 is in the form of a cylinder of uniform cross-section along its length. However, it is also possible for the penetrating part 24a to be tapered so as to have a decreasing cross-section whereby it forms a cone or truncated cone. It is also possible for the penetrating part to be triangular, square or other shape in cross-section, rather than have a circular cross-section. As will be appreciated, in such arrangements the bore 33 will have a shape generally complimentary to that of the penetrating part 24a of the heater so to maintain the functionality described above.

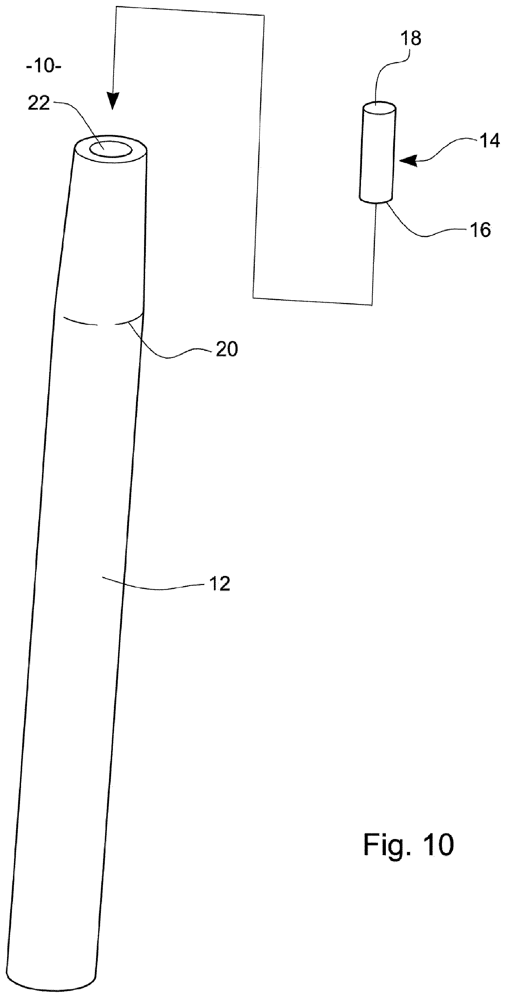

Figure 10 is an exploded perspective view illustration of a kit-of-parts for assembling an aerosol delivery system 10.

As will be appreciated, in the arrangements described above, the fluid-transfer article 34 is provided within a housing 32 of the aerosol carrier 14. In such arrangements, the housing of the carrier 14 serves to protect the aerosol precursor-containing fluid-transfer article, whilst also allowing the carrier 14 to be handled by a user without his/her fingers coming into contact with the aerosol precursor liquid retained therein.

Throughout this specification, including the claims which follow, unless the context requires otherwise, the words "have", "comprise", and "include", and variations such as "having", "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means, for example, +/- 10%.

The words "preferred" and "preferably" are used herein refer to embodiments of the invention that may provide certain benefits under some circumstances. It is to be appreciated, however, that other embodiments may also be preferred under the same or different circumstances. The recitation of one or more preferred embodiments therefore does not mean or imply that other embodiments are not useful, and is not intended to exclude other embodiments from the scope of the disclosure, or from the scope of the claims.

## Claims

1. An aerosol delivery system (10) comprising:
an aerosol-generation apparatus (12) comprising:
a heater (24), the heater comprising a base part and a penetrating part (24a) extending from the base part;
a carrier (14) having a first housing (32) containing a fluid-transfer article (34); and
a further housing (37) supporting said heater (24), said first housing (32) and said further housing (37) being separable,
wherein:
the fluid-transfer article (34) is for holding an aerosol precursor and transferring the aerosol precursor to an activation surface (34b) of said fluid-transfer article (34),
the fluid-transfer article (34) has a bore (33) therein within which there is at least the penetrating part (24a) of the heater (24), the wall of the bore (33) surrounding the penetrating part (24a) of the heater (24) and forming said activation surface (34b),
the activation surface (34b) has a varying spacing from said penetrating part (24a), thereby to define at least one air-flow pathway between said activation surface (34b) and said penetrating part (24a) of said heater (24), and
the fluid-transfer article (34) includes a first region (34) surrounding said penetrating part (24a) of said heater (24), a surface of said first region (34) forming said activation surface (34b);
wherein said further housing (37) has an inlet (50), said at least one air-flow pathway extending from said inlet (50), and said first housing (32) has an outlet, said air-flow pathway extending to said outlet;
wherein,
when the first housing (32) and the further housing (37) are fitted together, there is a gap (38) between an end (16) of the carrier (14) and the heater (24),
wherein the gap (38) forms part of, or defines, the at least one airflow pathway between the inlet (50) and the variable spacing; and
wherein the base part of the heater (24) forms a bore which bounds the gap (38) so as to facilitate the heating of the air before it reaches the variable spacing.

2. An aerosol delivery system (10) according to claim 1, wherein at least one part of the wall of the bore (33) is in contact with said penetrating part (24a) of said heater (24), and at least another part of said bore (33) defines at least one channel (35) between the wall of the bore (33) and the penetrating part (24a) of the heater (24), said at least one air-flow pathway being along said at least one channel (35).

3. An aerosol delivery system (10) according to claim 2, wherein the aerosol-generation apparatus (12) has a plurality of said channels (35) around said penetrating part (24a) of the heater (24).

4. An aerosol delivery system (10) according to claim 2 or claim 3, wherein said penetrating part (24a) is elongate in the direction of the axis of the bore (33).

5. An aerosol delivery system (10) according to claim 4, wherein said at least one channel (35) extends in a direction parallel to the direction of elongation of said penetrating part (24a) of said heater (24).

6. An aerosol delivery system (10) according to any one of the preceding claims, wherein said penetrating part (24a) of said heater (24) is cylindrical.

7. An aerosol delivery system (10) according to any one of the preceding claims, wherein the inlet (50) comprises two or more inlets.

8. An aerosol delivery system (10) according to any one of the preceding claims, wherein the gap (38) comprises plural gaps.

9. An aerosol delivery system (10) according to any one of the preceding claims, wherein the further housing (37) is integral with a housing (26) of the aerosol-generation apparatus (12) containing an electrical energy supply (28) of the aerosol-generation apparatus.

10. An aerosol delivery system (10) according to claim 9, the housing (26, 37) which contains the electrical energy supply (28) further containing: control circuitry operative by a user or upon detection of air and/or vapour being drawn into the aerosol-generation apparatus (12) through air-intake apertures (20); and a coupling (30) for electrically coupling the electric energy supply (28) to control circuitry for powering and controlling operation of the heater (24).

11. An aerosol delivery system (10) according to claim 9 or 10, wherein the housing (26, 37) which contains the electrical energy supply (28) defines a receptacle (22) for receiving the carrier (14).

12. An aerosol delivery system (10) according to any one of the preceding claims, wherein the carrier (14) has a first end (16) and a second end (18); wherein, when the first housing (32) and the further housing (37) are fitted together, the gap (38) is between the first end (16) of the carrier (14) and the heater (24), and the second end (18) extends from the aerosol-generation apparatus (12) for insertion into a user's mouth.

## Patentansprüche

1. Aerosolabgabesystem (10), umfassend:
eine Aerosolerzeugungsvorrichtung (12), umfassend:
eine Heizvorrichtung (24), wobei die Heizvorrichtung einen Basisteil und einen Eindringteil (24a), der sich von dem Basisteil weg erstreckt, umfasst;
einen Träger (14) mit einem ersten Gehäuse (32), das einen Fluidtransportartikel (34) enthält, und einem weiteren Gehäuse (37), das die Heizvorrichtung (24) trägt, wobei das erste Gehäuse (32) und das weitere Gehäuse (37) trennbar sind,
wobei:
der Fluidtransportartikel (34) dem Speichern eines Aerosolvorläufers und Transportieren des Aerosolvorläufers zu einer Aktivierungsfläche (34b) des Fluidtransportartikels (34) dient,
der Fluidtransportartikel (34) eine Bohrung (33) darin aufweist, innerhalb derer zumindest der Eindringteil (24a) der Heizvorrichtung (24) angeordnet ist, wobei die Wand der Bohrung (33) den Eindringteil (24a) der Heizvorrichtung (24) umgibt und die Aktivierungsoberfläche (34b) ausbildet,
die Aktivierungsfläche (34b) einen variierenden Abstand von dem Eindringteil (24a) aufweist, wodurch zumindest ein Luftströmungspfad zwischen der Aktivierungsfläche (34b) und dem Eindringteil (24a) der Heizvorrichtung (24) definiert ist, und
der Fluidtransportartikel (34) eine erste Region (34) umfasst, welche den Eindringteil (24a) der Heizvorrichtung (24) umgibt, wobei eine Fläche der ersten Region (34) die Aktivierungsfläche (34b) ausbildet;
wobei das weitere Gehäuse (37) einen Einlass (50) aufweist, wobei der zumindest eine Luftströmungspfad sich von dem Einlass (50) weg erstreckt, und das erste Gehäuse (32) einen Auslass aufweist, wobei der Luftströmungspfad sich bis zu dem Auslass erstreckt;
wobei, wenn das erste Gehäuse (32) und das weitere Gehäuse (37) zusammengefügt sind, ein Spalt (38) zwischen einem Ende (16) des Trägers (14) und der Heizvorrichtung (24) ist,
wobei der Spalt (38) einen Teil des zumindest einen Luftströmungspfads zwischen dem Einlass (50) und dem variablen Abstand ausbildet oder definiert; und
wobei der Basisteil der Heizvorrichtung (24) eine Bohrung ausbildet, welche den Spalt (38) eingrenzt, um das Erhitzen der Luft, bevor sie den variablen Abstand erreicht, zu erleichtern.

2. Aerosolabgabesystem (10) nach Anspruch 1, wobei zumindest ein Teil der Wand der Bohrung (33) in Kontakt mit dem Eindringteil (24a) der Heizvorrichtung (24) ist und zumindest ein anderer Teil der Bohrung (33) zumindest einen Kanal (35) zwischen der Wand der Bohrung (33) und dem Eindringteil (24a) der Heizvorrichtung (24) definiert, wobei der zumindest eine Luftströmungsweg entlang des zumindest einen Kanals (35) verläuft.

3. Aerosolabgabesystem (10) nach Anspruch 2, wobei die Aerosolerzeugungsvorrichtung (12) eine Vielzahl von Kanälen (35) rund um den Eindringteil (24a) der Heizvorrichtung (24) aufweist.

4. Aerosolabgabesystem (10) nach Anspruch 2 oder 3, wobei der Eindringteil (24a) in Richtung der Achse der Bohrung (33) länglich ist.

5. Aerosolabgabesystem (10) nach Anspruch 4, wobei der zumindest eine Kanal (35) sich in einer Richtung parallel zu der Längsrichtung des Eindringteils (24a) der Heizvorrichtung (24) erstreckt.

6. Aerosolabgabesystem (10) nach einem der vorangegangenen Ansprüche, wobei der Eindringteil (24a) der Heizvorrichtung (24) zylindrisch ist.

7. Aerosolabgabesystem (10) nach einem der vorangegangenen Ansprüche, wobei der Einlass (50) zwei oder mehrere Einlässe umfasst.

8. Aerosolabgabesystem (10) nach einem der vorangegangenen Ansprüche, wobei der Spalt (38) mehrere Spalte umfasst.

9. Aerosolabgabesystem (10) nach einem der vorangegangenen Ansprüche, wobei das weitere Gehäuse (37) einstückig mit einem Gehäuse (26) der Aerosolerzeugungsvorrichtung (12) ausgebildet ist, das eine elektrische Energieversorgung (28) der Aerosolerzeugungsvorrichtung enthält.

10. Aerosolabgabesystem (10) nach Anspruch 9, wobei das Gehäuse (26, 37), welches die elektrische Energieversorgung (28) enthält, ferner Folgendes enthält: eine Steuerschaltanordnung, die durch einen Benutzer oder beim Detektieren eines Einsaugens von Luft und/oder Dampf in die Aerosolerzeugungsvorrichtung (12) durch Luftansaugöffnungen (20) betätigbar ist; und eine Kopplung (30) zum elektrischen Koppeln der elektrischen Energieversorgung (28) zum Steuern einer Schaltanordnung zum Stromversorgen und Steuern des Betriebs der Heizvorrichtung (24).

11. Aerosolabgabesystem (10) nach Anspruch 9 oder 10, wobei das Gehäuse (26, 37), welches die elektrische Energieversorgung (28) enthält, einen Behälter (22) zum Aufnehmen des Trägers (14) definiert.

12. Aerosolabgabesystem (10) nach einem der vorangegangenen Ansprüche, wobei der Träger (14) ein erstes Ende (16) und ein zweites Ende (18) aufweist; wobei, wenn das erste Gehäuse (32) und das weitere Gehäuse (37) zusammengefügt sind, der Spalt (38) zwischen dem ersten Ende (16) des Trägers (14) und der Heizvorrichtung (24) ist und das zweite Ende (18) sich von der Aerosolerzeugungsvorrichtung (12) weg zum Einbringen in den Mund eines Benutzers erstreckt.

## Revendications

1. Système de distribution d'aérosol (10), comprenant :
un appareil de génération d'aérosol (12), comprenant :
un dispositif de chauffage (24), le dispositif de chauffage comprenant une partie de base et une partie pénétrante (24a) s'étendant à partir de la partie de base ;
un support (14) présentant un premier logement (32) contenant un article de transfert de fluide (34) ; et
un logement supplémentaire (37) supportant ledit dispositif de chauffage (24), ledit premier logement (32) et ledit logement supplémentaire (37) pouvant être séparés,
dans lequel :
l'article de transfert de fluide (34) est destiné à contenir un précurseur d'aérosol et à transférer le précurseur d'aérosol vers une surface d'activation (34b) dudit article de transfert de fluide (34),
l'article de transfert de fluide (34) présente un alésage (33) en son sein à l'intérieur duquel se trouve au moins la partie pénétrante (24a) du dispositif de chauffage (24), la paroi de l'alésage (33) entourant la partie pénétrante (24a) du dispositif de chauffage (24) et formant ladite surface d'activation (34b), la surface d'activation (34b) présente un espacement variable par rapport à ladite partie pénétrante (24a), en définissant ainsi au moins un trajet d'écoulement d'air entre ladite surface d'activation (34b) et ladite partie pénétrante (24a) dudit dispositif de chauffage (24), et
l'article de transfert de fluide (34) inclut une première région (34) entourant ladite partie pénétrante (24a) dudit dispositif de chauffage (24), une surface de ladite première région (34) formant ladite surface d'activation (34b) ;
dans lequel ledit logement supplémentaire (37) présente une entrée (50), ledit au moins un trajet d'écoulement d'air s'étendant depuis ladite entrée (50), et ledit premier logement (32) présente une sortie, ledit trajet d'écoulement d'air s'étendant vers ladite sortie ;
dans lequel
lorsque le premier logement (32) et le logement supplémentaire (37) sont montés ensemble, il existe un espace (38) entre une extrémité (16) du support (14) et le dispositif de chauffage (24),
dans lequel l'espace (38) fait partie de ou définit le au moins un trajet d'écoulement d'air entre l'entrée (50) et l'espacement variable ; et
dans lequel la partie de base du dispositif de chauffage (24) forme une ouverture qui délimite l'espace (38) de manière à faciliter le chauffage de l'air avant qu'il n'atteigne l'espacement variable.

2. Système de distribution d'aérosol (10) selon la revendication 1, dans lequel au moins une partie de la paroi de l'alésage (33) est en contact avec ladite partie pénétrante (24a) dudit dispositif de chauffage (24), et au moins une autre partie dudit alésage (33) définit au moins un canal (35) entre la paroi de l'alésage (33) et la partie pénétrante (24a) du dispositif de chauffage (24), ledit au moins un trajet d'écoulement d'air étant le long dudit au moins un canal (35).

3. Système de distribution d'aérosol (10) selon la revendication 2, dans lequel l'appareil de génération d'aérosol (12) présente une pluralité desdits canaux (35) autour de ladite partie pénétrante (24a) du dispositif de chauffage (24).

4. Système de distribution d'aérosol (10) selon la revendication 2 ou la revendication 3, dans lequel ladite partie pénétrante (24a) est allongée dans la direction de l'axe de l'alésage (33).

5. Système de distribution d'aérosol (10) selon la revendication 4, dans lequel ledit au moins un canal (35) s'étend dans une direction parallèle à la direction d'allongement de ladite partie pénétrante (24a) dudit dispositif de chauffage (24).

6. Système de distribution d'aérosol (10) selon l'une quelconque des revendications précédentes, dans lequel ladite partie pénétrante (24a) dudit dispositif de chauffage (24) est cylindrique.

7. Système de distribution d'aérosol (10) selon l'une quelconque des revendications précédentes, dans lequel l'entrée (50) comprend deux entrées ou plus.

8. Système de distribution d'aérosol (10) selon l'une quelconque des revendications précédentes, dans lequel l'espace (38) comprend plusieurs espaces.

9. Système de distribution d'aérosol (10) selon l'une quelconque des revendications précédentes, dans lequel le logement supplémentaire (37) est solidaire d'un logement (26) de l'appareil de génération d'aérosol (12) contenant une alimentation en énergie électrique (28) de l'appareil de génération d'aérosol.

10. Système de distribution d'aérosol (10) selon la revendication 9, le logement (26, 37) qui contient l'alimentation en énergie électrique (28) contenant en outre :
des circuits de commande fonctionnant par l'intermédiaire d'un utilisateur ou lors de la détection d'air et/ou de vapeur aspiré(e) dans l'appareil de génération d'aérosol (12) à travers des ouvertures d'admission d'air (20) ; et
un couplage (30) pour coupler électriquement l'alimentation en énergie électrique (28) aux circuits de commande afin d'alimenter et de commander le fonctionnement du dispositif de chauffage (24).

11. Système de distribution d'aérosol (10) selon la revendication 9 ou 10, dans lequel le logement (26, 37) qui contient l'alimentation en énergie électrique (28) définit un réceptacle (22) pour recevoir le support (14).

12. Système de distribution d'aérosol (10) selon l'une quelconque des revendications précédentes, dans lequel le support (14) présente une première extrémité (16) et une seconde extrémité (18) ; dans lequel lorsque le premier logement (32) et le logement supplémentaire (37) sont ajustés ensemble, l'espace (38) est entre la première extrémité (16) du support (14) et le dispositif de chauffage (24), et la seconde extrémité (18) s'étend à partir de l'appareil de génération d'aérosol (12) pour être insérée dans la bouche d'un utilisateur.
